(19) Europäisches Patentamt
European Patent Office
Office européen des brevets

(11) Numéro de publication: **0 141 711**
**A2**

# (12) DEMANDE DE BREVET EUROPEEN

(21) Numéro de dépôt: **84402068.5**

(22) Date de dépôt: **15.10.84**

(51) Int. Cl.[4]: **G 01 N 33/545**, G 01 N 33/80, C 12 Q 1/56

(30) Priorité: **13.10.83 FR 8316302**

(43) Date de publication de la demande: **15.05.85**
**Bulletin 85/20**

(84) Etats contractants désignés: **AT BE CH DE GB IT LI LU NL SE**

(71) Demandeur: **CHOAY S.A., 48, Avenue Théophile-Gautier, F-75782 Paris Cédex 16 (FR)**
Demandeur: **CENTRE NATIONAL DE TRANSFUSION SANGUINE, 8, rue Alexandre Cabanel, F-75732 Paris Cédex 15 (FR)**

(72) Inventeur: **Faure, Alain, Avenue du Colonel Bonnet, F-75016 Paris (FR)**
Inventeur: **Ropars, Claude, 6, rue Toulouse Lautrec, F-37000 Tours (FR)**
Inventeur: **Doinel, Christian, 6, avenue du Béarn, F-78310 Maurepas (FR)**
Inventeur: **Lefrancier, Pierre, 46, allée de la Mare l'Oiseau Chevry II, F-91190 Gif-sur-Yvette (FR)**
Inventeur: **Maman, Michel, 2, rue Carves, F-92120 Montrouge (FR)**
Inventeur: **Level, Michel, 24, rue Sibuet, F-75012 Paris (FR)**

(74) Mandataire: **Peaucelle, Chantal et al, Cabinet Plasseraud 84, rue d'Amsterdam, F-75009 Paris (FR)**

(54) **Nouveaux conjugués élaborés par fixation d'un ligand sur un support insoluble, leur préparation et leurs applications biologiques.**

(57) Les conjugués sont élaborés à partir de supports greffés et d'une molécule à activité biologique renfermant au moins un motif saccharidique ou du type des glucosamino-glucuroglycanes.

EP 0 141 711 A2

"Nouveaux conjugués élaborés par fixation d'un ligand sur un support insoluble, leur préparation et leurs applications biologiques".

L'invention est relative à de nouveaux conjugués élaborés par fixation d'une molécule organique, ou ligand, sur un support insoluble, à leur préparation et à leurs applications biologiques.

Les conjugués du type support-ligand sont largement utilisés en biologie, plus particulièrement pour extraire sélectivement des macromolécules d'origine naturelle à partir d'un milieu d'extraction complexe.

En effet, par fixation sur un support insoluble, inerte, d'un ligand qui sera reconnu spécifiquement par la substance biologique à extraire dans un milieu biologique donné, il est possible de retenir sélectivement ladite substance puis, par élution, de l'obtenir à un degré de pureté élevée.

Cette purification effectuée selon le principe bien connu de la chromatographie d'affinité, présente un grand intérêt pour obtenir des molécules aussi diverses que des anticorps, des enzymes, des inhibiteurs d'enzymes, des lectines, des récepteurs hormonaux et des acides nucléiques.

Il est cependant nécessaire de disposer de supports ne donnant pas lieu à des fixations non spécifiques, dues par exemple à des liaisons de type hydrophobe ou ionique, pouvant être substituées, quantitativement et de manière reproductible par un ligand et présentant les qualités requises pour une application donnée.

Ainsi, pour les nombreuses utilisations nécessitant la mise en contact des conjugués avec le sang, on mesurera l'intérêt de disposer de supports hémocompatibles.

Outre ces qualité, on conçoit que pour des utilisations à grande échelle, il est important de disposer de conjugués de faible coût de revient.

Or, les supports proposés à ce jour pour l'élaboration de tels conjugués ne répondent pas de manière totalement satisfaisante aux diverses exigences exprimées ci-dessus, en particulier au niveau des problèmes de la spécificité de la fixation des macromolécules à retenir, de celui de l'hémocompatibilité et du prix de revient.

Les travaux des inventeurs dans ce domaine les ont amenés à étudier les possibilités d'utilisation de divers supports, plus spécialement, ceux du type supports greffés.

De tels supports sont décrits dans la demande de brevet FR n° 82 17 315 du 15.10.1982, au nom du COMMISSARIAT A L'ENERGIE ATOMIQUE.

Il s'agit de supports particulaires greffés en surface, c'est-a-dire obtenus par greffage sur des particules d'un polymère insoluble de molécules insaturées capables de se polymériser et portant une fonction organique substituable du type allylamine, acrylamide ou acide acrylique.

D'une manière générale, les particules de copolymère sont insolubles dans la plupart des solvants et notamment dans l'eau et les solutions aqueuses. Elles sont inertes dans les conditions de mise en oeuvre et possèdent un taux de gonflement faible, une densité supérieure à l'unité, une bonne tenue dimensionnelle sous pression et une bonne résistance à l'écrasement.

A l'étude de ces supports, en vue plus spécialement de réaliser des immunoabsorbants hémocompatibles présentant une forte capacité d'absorbtion très spécifique vis à vis des anticorps anti-groupes sanguins A et B, immunoabsorbants portant le déterminant trisaccharidique responsable de l'antigénicité du groupe sanguin A ou celui du groupe B, il est apparu que seuls certains d'entre eux étaient utilisables. Les travaux effectués ont alors montré qu'en modifiant leur groupe fonctionnel terminal, les motifs greffés de ces supports pouvaient être utilisés pour former en partie le bras espaçant le ligand du support, la nécessité de ce bras étant bien connue en chromatographie d'affinité.

Les résultats obtenus concernant ces immunoabsorbants ont ensuite amené à développer, par couplage, avec ou sans bras additionnels, sur ces supports, de nouveaux conjugués renfermant des substances biologiques comportant comme ces déterminants des motifs de glucosaminoglucuroglycanes, c'est-à-dire des motifs sucres aminés du type glucosamine et/ou des motifs acide uronique, conjugués élaborés à partir de supports greffés et de ligands à activité biologique.

Elle vise plus spécialement à fournir des conjugués de ce type hémocompabtbles et possédant des capacités d'absorption spécifique élevée.

L'invention vise également à fournir un procédé de préparation de ces conjugués de mise en oeuvre aisée.

Elle vise, en outre, les applications biologiques de ces conjugués en particulier, dans des opérations d'épuration sanguine et de purification de substances biologiques.

Les conjugués de l'invention sont caractérisés en ce qu'ils sont élaborés à partir de supports greffés tels qu'évoqués ci-dessus et d'une molécule fixée de façon covalente au support, possédant une activité biologique. Cette molécule fixée renferme au moins un motif saccharidique ou une structure plus complexe du type des glucosaminoglucuroglycanes et est choisie en particulier, parmi les déterminants antigéniques de groupe sanguin, l'héparine, des substrats biologiques, tels que des cellules ou des membranes cellulaires, en particulier, des stromas d'érythrocytes, la N-acétylglusosamine ou un dérivé de N-acétylglucosamine.

La molécule en question est reliée le cas échéant, au support par l'intermédiaire d'une chaîne jouant le rôle de bras espaceur. Ce bras peut être formé à l'aide, d'une part, des motifs du monomère greffé sur le support et, d'autre part, de la molécule considérée et/ou allongé ou fourni par un composé chimique mis en oeuvre pour le couplage.

Ces conjugués permettent de retenir avec une haute spécificité, à partir d'un milieu complexe et hétérogène, les substances ayant une affinité pour la molécule fixée en question.

Il est également possible, si on le souhaite de récupérer, par exemple, par élution, la substance fixée que l'on obtient à un degré de pureté élevée.

Selon une disposition préférée de l'invention, le bras espaceur comporte un groupe hydrazide. Ce groupe est avantageusement obtenu directement à partir de supports greffés à l'aide de motifs monomères à fonction acrylamide, par transformation en fonction acrylhydrazide.

Selon une autre disposition préférée, avantageusement utilisée en combinaison avec celle qui précède, le taux de greffage des supports entrant dans la constitution des conjugués est de l'ordre d'environ moins de 10 %, en particulier, d'environ moins de 7 %. Des taux de greffages préférés, en particulier, avec des motifs acrylamide sont de l'ordre de 1 à 5 % plus spécialement voisins de 2 à 3 %.

D'une manière générale, les conjugués résultants sont avantageusement hémocompatibles ce qui permet d'éviter des fixations non spécifiques et/ou des perturbations biologiques dans des applications pour des épurations sanguines, avec restitution du milieu épuré.

Des conjugués préférés à cet égard renferment, comme matière polymère de base constituant le support, du polychlorure de vinyle ou PVC.

Un groupe préféré de conjugués de l'invention, utilisable comme immunoabsorbant, comprend, comme molécule fixée, un déterminant antigénique de groupe sanguin.

Des conjugués préférés renfermant le déterminant antigénique B répondent à la structure <u>a</u>.

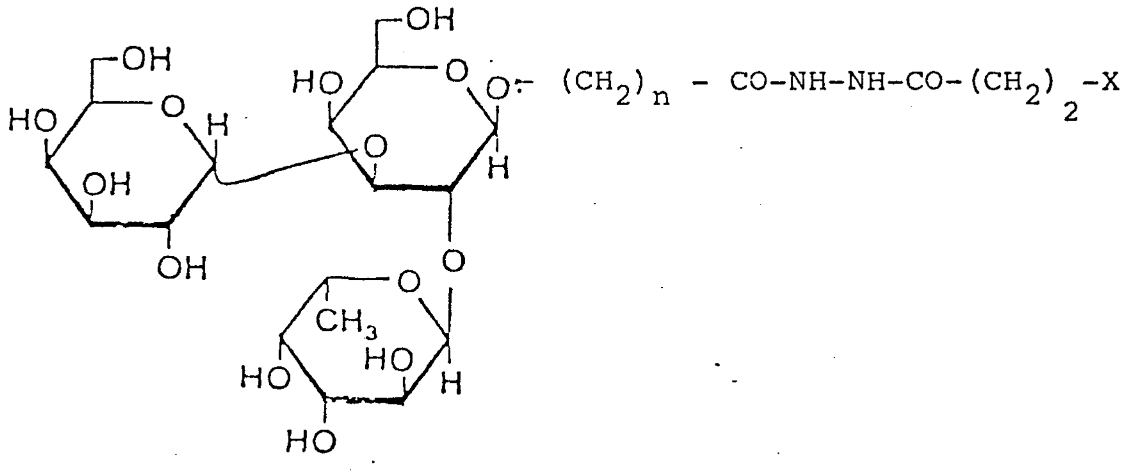

avec n représentant un nombre de à et X représentant le restant du bras fourni par le support greffé et ce support de base lui-même.

D'autres conjugués préférés répondent à la même structure mais comportent le déterminant antigénique A.

Dans un autre groupe préféré de conjugués de l'invention, la molécule fixée est formée par l'héparine. De tels produits sont particulièrement intéressants pour effectuer, à partir du sang, plus spécialement des épurations de certains types de lipoprotéines, en particulier de LDL (lipoprotéines à faible densité).

Dans ces conjugués, l'héparine peut être fixée directement sur le support ou par l'intermédiaire d'un bras.

Des bras du type diaminohexane s'avèrent appropriés.

Un autre groupe de conjugués renferme des substrats biologiques tels que des stromas d'érythrocytes.

Ces conjugués permettent, notamment, d'effectuer des épurations et vivo dans un contexte pathologique lié à la présence d'anticorps reconnaissant des antigènes de groupe sanguin.

Dans un autre groupe préféré présentant un grand intérêt pour la purification de lectines ou de substances, telles que les enzymes glycolytiques, dotées d'une affinité

spécifique pour la N-acétyl glucosamine ou un autre motif saccharidique, l'haptène fixé sur le support est constitué par la N-acétyl glucosamine ou le motif saccharidique.

Des conjugués préférés de ce groupe répondent à la structure b.

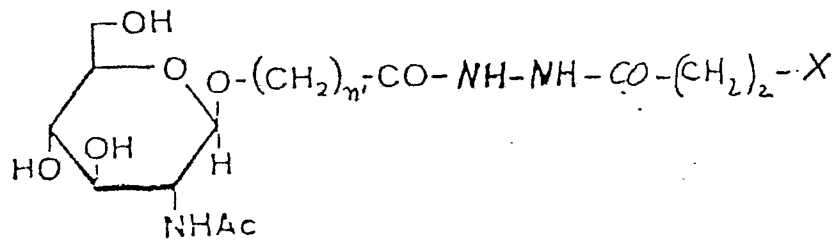

dans laquelle n' est un nombre de 2 à 8, Ac représente le groupe acétyle et X est tel que défini ci-dessus.

Les conjugués de l'invention sont préparés par mise en contact de la molécule biologiquement active, avec le support greffé, avantageusement activé selon les techniques classiques, et le cas échéant après avoir subi un traitement afin de modifier les groupes fonctionnels en bout de chaîne.

Pour la préparation des conjugués renfermant un déterminant antigénique de groupe sanguin, on a avantageusement recours à un support greffé à l'aide de motifs acrylamide puis soumis à une hydrazinolisis afin de former des groupements acrylhydrazide.

L'hydrazine n'ayant pas réagi est éliminée également par lavage.

Le support ainsi modifié est directement utilisable pour le couplage avec un dérivé approprié de déterminant antigénique.

Un tel support est avantageusement hémocompatible et par ailleurs ne donne pas lieu à des absorptions non spécifiques.

On peut l'utiliser dans des systèmes avec restitution du milieu biologique (plasma sanguin) après épuration

externe.

Grâce à cette modification de la fonctionnalité du polymère greffé, à la fois le support et le déterminant antigénique participant à la formation du bras de couplage qui doit assurer un espacement entre les deux entités du conjugué.

Le dérivé du déterminant antigénique comprend alors conformément à l'invention une chaîne terminée par une fonction carboxyle de formule $O-(CH_2)_n-COOH$ dans laquelle n est avantageusement un nombre de 2 à 4, les groupes -OH du déterminant étant, par ailleurs, bloqués par des groupements protecteurs tels qu'utilisés dans la chimie des sucres.

Le couplage est réalisé de manière progressive, sous agitation, de préférence à température ambiante.

Le dérivé du déterminant antigénique mis en oeuvre est au préalable avantageusement activé.

Des solvants du type du diméthylformamide DMF constituent des milieux appropriés.

Avantageusement, on utilise des quantités équimolaires de déterminant antigénique, de N-méthylmorpholine et de chloroformiate d'alkyle.

Compte tenu de l'hétérogénéité du milieu réactionnel et de la forte réactivité chimique des agents couplants, il apparaît souhaitable d'utiliser un volume de milieu d'activation équivalent à celui du support humide.

De plus, il est souhaitable de soumettre le support hydrazide à un dégazage avant d'effectuer le couplage.

Les déterminants antigéniques A ou B comportant une chaîne de substitution à terminaison carboxyle ci-dessus constituent des produits nouveaux et, en tant que tels, entrent également dans le cadre de l'invention.

Ces dérivés sont avantageusement obtenus à partir des dérivés correspondants dans lesquels tous les groupes

-OH sont bloqués par des groupes protecteurs du type benzyle, benzoyle, acétyle, ou pour deux groupements voisins benzylidène et la chaîne de substitution est constituée par un groupe $-O-(CH_2)_{n-1}-OH$.

Conformément à un mode préféré de réalisation de l'invention, ces dérivés sont alors soumis à un traitement d'oxydation puis d'hydrogénation et de saponification.

La réaction d'oxydation est avantageusement réalisée à l'aide d'un agent oxydant tel que l'anhydride chromique. Cette étape est avantageusement réalisée à température ambiante dans un milieu solvant du type de ceux renfermant en mélange le dichlorométhane et la pyridine.

La chaîne à terminaison aldéhyde qui se forme intermédiairement est transformée en chaîne $-O-(CH_2)_2-CH=CH$ COOMe sous l'action d'un traitement du type de celui effectué à l'aide du réactif de Wittig. Dans ce cas, la réaction est réalisée à une température voisine de 100°C notamment de 90°C.

La réaction d'hydrogénation est avantageusement effectuée en présence de catalyseur, ce qui permet de libérer les groupes -OH de leurs groupements protecteurs hydrogénolysables et de transformer la chaîne de substitution en groupe $-O-(CH_2)_n-COOMe$.

Par saponification de l'ester méthylique, on obtient alors la chaîne à terminaison carboxyle recherchée.

Les conjugués renfermant des stromas d'érythrocytes sont avantageusement préparés par mise en contact d'un support greffé préalablement lavé et activé par le glutaraldéhyde et des stromas, également soumis à un prélavage. Le couplage est de préférence réalisé à basse température, plus spécialement entre 0 et 10°C environ, avantageusement à environ 4°C.

Les groupes fonctionnels libres éventuellement présents sur le support peuvent être bloqués si nécessaire

à l'aide de réactifs appropriés.

Des supports du type PVC-acrylamide ou PVC-acide acrylique permettent d'obtenir des conjugués satisfaisants au regard, en particulier, de leur spécificité d'absorption, les supports hémocompatibles, plus spécialement ceux comportant des motifs acrylamide étant préférés dans les applications pour des épurations sanguines.

D'autres conjugués de l'invention du type de ceux renfermant un dérivé de N-acétylglucosamine fixé sur un support greffé sont obtenus par couplage sur le support activé d'un dérivé approprié de la N-acétylglucosamine.

Selon un mode préféré de réalisation de l'invention, on utilise un support greffé à l'aide de motifs acrylamide avantageusement à raison d'environ moins de 10 %, notamment de 1 à 7 %, ces motifs étant soumis à un traitement d'hydrazinolyse comme indiqué ci-dessus afin de former des groupes acrylhydrazide.

Le dérivé de N-acétyl glucosamine comporte un bras de substitution, avantageusement en position 1, à terminaison carboxyle.

Comme indiqué ci-dessus, les conjugués de l'invention présentent des capacités d'absorption hautement spécifiques qui leur confèrent un grand intérêt pour de nombreuses applications biologiques.

Ils sont utilisables, en particulier, comme immunoabsorbants pour épurer le plasma des substances reconnaissant spécifiquement l'haptène ou le ligand des conjugués.

Ils permettent ainsi d'effectuer des épurations thérapeutiques dans un contexte pathologique.

L'utilisation d'immunoabsorbants renfermant de l'héparine permet, par ailleurs, d'éliminer sélectivement comme déjà indiqué des lipoprotéines LDL.

Les conjugués ayant une activité de groupe sanguin par l'intermédiaire des stromas ou des oligosaccharides

fixés, et ceux comportant des oligosaccharides tels que la N-acétylglucosamine, permettent l'obtention de réactifs biologiques spécifiques, utilisables pour le typage des groupes sanguins (Rhésus A, B, O et autres), par exemple, par agglutination, l'étude structurale de membranes cellulaires normales ou transformées, la séparation et/ou l'identification de cellules lymphotateuses, l'analyse et la purification de glucoprotéines et de polysàccharides.

<u>EXEMPLE 1</u> : <u>Préparation d'un conjugué renfermant le déterminant antigénique du groupe sanguin B fixé sur un support polychlorure de vinyle-polyacrylamide.</u>

Pour préparer ce conjugué, on met en oeuvre :

- d'une part un dérivé du trisaccharide B de formule (1) :

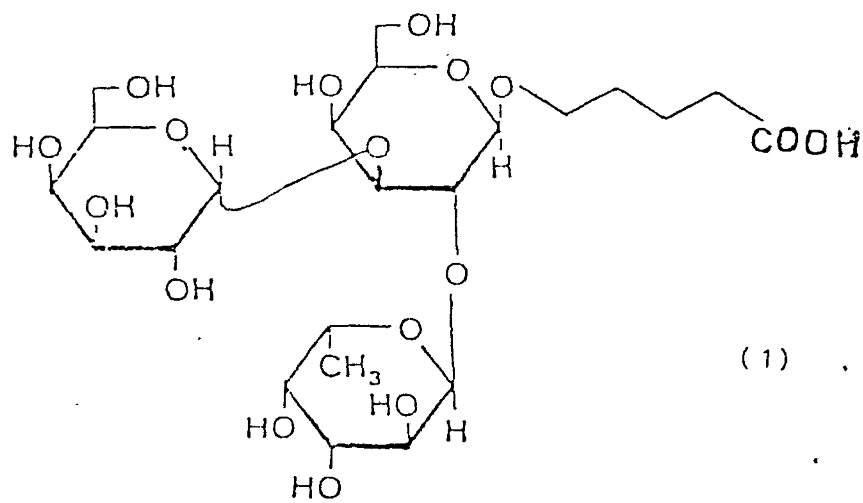

(1)

dans laquelle le symbole 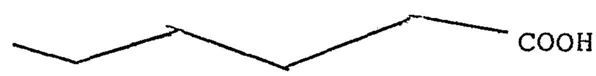 représente une chaîne $-CH_2-CH_2-CH_2-CH_2-COOH$

- d'autre part, un support répodnant aux caractéristiques suivantes :

- taux de greffage en acrylamide en poids : 12,5 % (ce qui représente une teneur de 1,86 mmole d'équivalent amidé par gramme de gel, soit la capacité potentielle de couplage au gel) ;

- taux de greffage en acrylamide dosé : 2,35 % ;
- taux de gonflement dans l'eau (25°C) : 4,25 % ;
- volume spécifique : 2,2 ml/g.

Cette préparation est effectuée selon les étapes a et b suivantes :

a. hydranolyse du support PVC-acrylamide,

b. couplage du trisaccharide 1 sur le support PVC-acrylhydrazide.

On réalise chacune de ces étapes comme suit :

a. hydrazinolyse.

On lave plusieurs fois à l'eau distillée un échantillon de support. Après chaque lavage, on laisse le gel sédimenter et on laisse décanter la partie surnageante. Le gel lavé est laissé dans l'eau durant 24 heures.

On introduit 5,5 ml (2,5 g) de gel ainsi traité dans un réacteur en présence d'une solution aqueuse 12 M d'hydrazine.

Le mélange est soumis à agitation durant 24 heures à température ambiante à l'aide d'un turbula, puis est filtré. On effectue ensuite plusieurs lavages avec une solution de NaCl 0,2 M jusqu'à l'absence d'hydrazine libre. On vérifie l'absence d'hydrazine dans les éluats de lavage par addition d'une solution aqueuse de trinitrobenzènesulfonate à 3 % (on ne doit pas obtenir de coloration rouge).

Le gel est ensuite rincé plusieurs fois à l'eau distillée.

b. couplage du trisaccharide 1 sur le support.

Le gel hydrazide est préalablement dégazé. On solubilise 46,75 μmoles du trisaccharide 1 dans 5,5 ml de DMF anhydre en présence de 6,75 μmoles (≃ 6 μl) de N-méthyl-morpholine à -15°C.

On ajoute 46,75 μmoles (≃ 6 μl) de chloroformiate d'isobutyle. Le mélange est incubé pendant 5 minutes à -15°C

puis ajouté à 2,5 g (soit 5,5 ml) de support PVC-hydrazide rincé, contenu dans le réacteur.

La suspension est mise en agitation rotative pendant 30 minutes à température ambiante.

On lave le mélange à l'aide de 3 volumes de tampon borate 0,2 M (pH 8) de façon à éliminer HCl formé. Le gel est ensuite rincé à l'eau distillée jusqu'à neutralité de l'éluat.

On le conserve en suspension dans l'eau à 4°C et à l'abri de la lumière pour éviter le développement bactérien à température ordinaire et les réactions d'oxydation dues à la lumière.

On obtient une fixation de 5,55 µmoles de trisaccharide <u>1</u> par gramme de support.

Dans un autre essai, en utilisant 31,17 µmoles de trisaccharide <u>1</u> par gramme de gel, on obtient une fixation de 4,13 µmoles/gramme de gel.

L'étude de l'absorption spécifique du conjugué trisaccharide-support contenant 5,5 µmoles de trisaccharide par gramme de gel vis-à-vis d'anti-sérum anti-B montre une absorption de 100 % d'anti-B. Les essais ont été réalisés en tubes sur 1 ml de conjugué en utilisant 2 ml d'anti-B (CNTS 5609, titre $C_{50}$ = 15,3) et 2 ml d'anti-A (CNTS 4941, titre $C_{50}$ = 14,2). Comme indiqué ci-dessus, l'absorption spécifique des anti-B s'élève à 100 %. L'absorption non spécifique d'anti-A est inférieure à 3 %.

Les résultats obtenus montrent la spécificité d'absorption du conjugué de l'invention vis-à-vis d'anticorps anti-B.

D'autres tests ont permis de vérifier l'absence d'absorption non spécifique sur les gels hydrazides non greffés.

l'invention fournit donc un système immunoabsorbant capable d'absorber sélectivement les anticorps dirigés

contre le déterminant antigénique correspondant au groupe sanguin de type B présent dans un sérum.

En opérant comme décrit précédemment, mais en utilisant à la place de la structure trisaccharidique du déterminant antigénique B la structure saccharidique du déterminant antigénique A, on obtient des immunoabsorbants capables d'absorber à 100 % les anticorps anti-groupe sanguin de type A.

Ces systèmes d'immunoabsorbants constituent donc un outil très précieux pour la transfusion sanguine en particulier pour l'obtention de composés permettant le typage spécifique de groupes sanguins ABO, Rhésus et autres.

EXEMPLE 2 : Préparation du trisaccharide de formule 1.

Le trisaccharide 1, mis en oeuvre pour la préparation du conjugué décrit dans l'exemple précédent, est avantageusement obtenu à partir du trisaccharide de formule (2) :

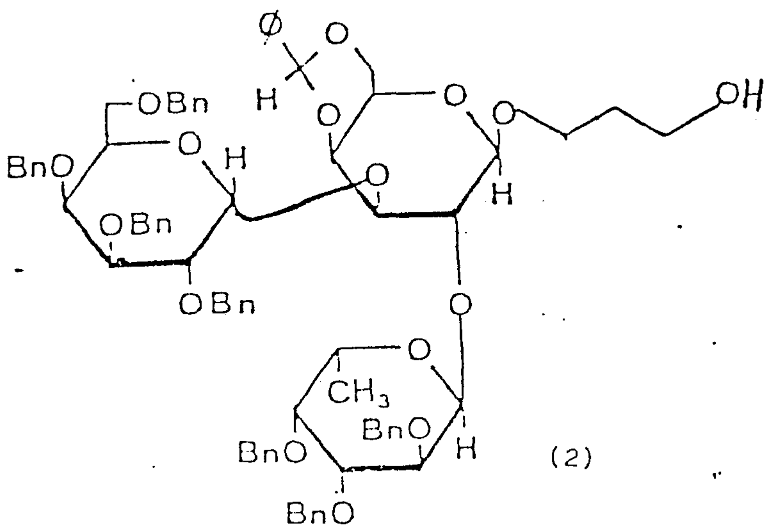

(2)

dans laquelle Ø représente un groupe $-C_6H_5$ et Bn un groupe benzyle.

La synthèse du trisaccharide 2 est rapportée dans l'exemple 3 de la demande de premier certificat d'addition N° 80 07614 à la demande de brevet FR N° 78 10558, ces demandes ayant été déposées respectivement le 3 Avril 1980

et le 10 Avril 1978 au nom de l'Agence Nationale de Valorisation de la Recherche.

A partir du trisaccharide 2, on effectue les étapes α à γ suivantes pour obtenir le trisaccharide 1.

α : oxydation du trisaccharide 2, ce qui conduit à l'obtention d'une chaîne $-CH_2-CH_2-CH = CH-COOCH_3$ à la place du groupe hydroxypropyle en position 1 du motif galactopyranose intermédiaire ;

β : hydrogénation du trisaccharide résultant, ce qui permet de libérer les groupes -OH des motifs saccharidiques et de conduire à une chaîne de substitution $-O-(CH_2)_4-COOCH_3$ dans la position 1 évoquée ci-dessus ;

γ : saponification du trisaccharide hydrogéné pour transformer le groupe ester de la chaîne de substitution en groupe acide.

Ces étapes sont réalisées comme suit :

α. oxydation.

Sous agitation, à la température ambiante, on prépare un mélange de 15 ml de dichlorométhane, de 0,644 ml de pyridine et de 400 mg d'anhydride chromique $CrO_3$.

Le mélange réactionnel est soumis à agitation durant 20 minutes et on ajoute alors en une seule fois une solution de 633 mg du dérivé 2 dans 5 ml de dichlorométhane.

Après agitation durant 20 minutes à la température ambiante, on extrait le mélange réactionnel avec du chloroforme (4 fois 20 ml).

La phase organique est lavée avec une solution de bicarbonate de sodium saturée, avec de l'eau puis séchée sur sulfate de sodium et évaporée.

Le résidu est élué sur une colonne de silice (10 g) à l'aide d'un mélange acétate d'éthyle-hexane 3:1 pour donner 576 mg d'aldéhyde, (rendement 91 % à partir de l'alcool) qui est immédiatement engagé dans la réaction suivante.

L'aldéhyde est dissous dans 15 ml de benzène anhydre et ajouté à 90°C en présence du réactif de Wittig $(C_6H_5)_3P = CH\text{-}COOCH_3$ pendant 2 h 30.

Le mélange est ensuite refroidi et évaporé à sec. Le produit est élué sur une colonne de gel de silice (50 g) à l'aide d'un mélange hexane-acétate d'éthyle 4:3 pour donner le composé insaturé renfermant la chaîne de substitution $-(CH_2)_2\text{-}CH{=}CH\text{-}COOCH_3$. On obtient 524 mg de produit, ce qui représente un rendement de 80 % par rapport à l'alcool de départ.

Le produit a un PF 152-153°C et un pouvoir rotatoire $[\alpha]_D^{20°C}$ égal à -3° dans le chloroforme.

Le spectre RMN et l'analyse centésimale sont en accord avec la structure attendue.

β. hydrogénation.

On ajoute une solution de 440 mg de trisaccharide dans 20 ml de méthanol sous hydrogène en présence de palladium sur charbon à 10 % 400 mg.

Après 48 heures, le catalyseur est essoré et le filtrat est évaporé pour donner le trisaccharide libre 188 mg 93 % sous forme d'une poudre blanche qui se décompose entre 110 et 114°. Le spectre de RMN et l'analyse centésimale sont en accord avec la structure attendue.

γ. saponification.

53 mg de produit sont purifiés sur une colonne de silice 60G par élution avec un mélange $CHCl_3/CH_3OH/CH_3COOH$ : 1/1/0,1.

Les éluats sont concentrés puis lyophilisés dans $CH_3\text{-}COOH$. On recueille 38 mg de produit qu'on redissout dans 0,5 ml d'eau. Après percolation sur résine AG 50 (5 ml)$WX_2$ dans l'eau, on effectue une lyophilisation. On récupère 35 mg (58 µmoles) de produit, 20 mg du produit purifié (33,2 µmoles) en solution dans 0,5 ml de méthanol sont incubés pendant 24 heures à température ordinaire en présence d'un volume de NaOH 0,1 N. On neutralise par la

résine DOWEX 50 $H^+$. On filtre et on effectue plusieurs lavages à l'eau. On concentre à sec le mélange réactionnel puis on le redissout dans l'eau et on le soumet à un traitement d'ultrafiltration puis à une lyophilisation. On obtient 20 mg de produit.

Caractéristiques analytiques : détermination de la pureté chimique par analyse en chromatographie phase gazeuse (méthode modifiée de Lamp et coll. 1963) ;
Pureté : 100 %
Rapport en nombre de motifs galactose au nombre de motifs fucose : 2,013.

EXEMPLE 3 : Fixation de stromas d'érythrocytes sur un support PVC-acide acrylique (taux de greffage %).

Une fixation irréversible de stromas d'érythrocytes humains sur un support PVC-acide acrylique est réalisée dans les conditions ci-dessous :

les stromas lyophilisés sont préparés selon la technologie de Faure A, Caron M, Leroy MH, Duval D, et Segard J, in Separation of cells and subcellular elements, éd. Peeters, Pergamon Press, N.Y. (1979), p. 99.

Avant utilisation, ils sont dissous sous agitation puis lavés en saline à basse force ionique.

Les microbilles greffées et lavées (600 mg) sont mises en suspension dans 15 ml d'une solution de 1-cyclohexyl-3-(2-morpholinoéthyl)carbodiimide-métho-p-toluène-sulfonate 95 % à 1 mg/ml et laissées sous agitation à température ambiante pendant 1 heure.

Les stromas lavés et décantés équivalant à 50 mg de stromas secs sont ajoutés à la suspension des microbilles du support traitées à la carbodiimide. L'ensemble est soumis à une agitation rotative à +4°C pendant une vingtaine d'heures. Les stromas non fixés sont éliminés par sédimentation et décantation des surnageants et 3 lavages avec NaCl 0,15M.

Le traitement à l'éthanolamine (1M en tampon bi-

carbonate 0,1 M, NaCl 0,15 M, pH 9,0) est pratiqué en flacons de verre à +4°C pendant 1 heure. Après lavages dans NaCl 0,15 M, les microbilles sont conservées à +4°C en tampons PBS, tween 1 %, albumine bovine 1 %, $NaN_3$ 0,5 %.

EXEMPLE 4 : Etude de l'incidence du taux de greffage sur la fixation des stromas et sur l'absorption des anticorps.

Des quantités identiques (600 mg) de microbilles greffées à 0,3 %, 0,4 %, 0,5 %, 2,7 %, 5 %, 7,2 % en acide acrylique puis activées par la carbodiimide (comme décrit dans l'exemple précédent) ont été parallèlement traitées avec l'équivalent de 10, 50, 100 mg de stromas secs, de phénotypes $A_1$, $R_1$, $R_2$.

Les tests d'absorption de 1 ml d'anti-sérum anti-A titrant manuellement le 64ème ont été pratiqués sur 100 mg de ces différents lots de billes traitées et recouvertes de stromas.

L'incubation est réalisée à température ambiante pendant 1 heure puis les surnageants, récupérés par centrifugation, sont testés selon la technique de Ropars C, Muller A, Hurel C et Leblanc J, Blood transfusion and immunohaemat XXIV N° 1 (1981).

Les résultats montrent que :

1°) Dans la série des microbilles traitées avec 10 mg de stromas pour 600 mg de billes, le pourcentage d'absorption d'activité anti-A s'échelonne de 80 % pour un taux de greffage de 0,4 %, jusqu'à 90 % pour un taux de greffage de 7,2 %.

2°) Dans les séries traitées par 50 mg et 100 mg de stromas, le pourcentage d'absorption est compris entre 88 et 93 % lorsque le taux de greffage est égal ou supérieur à 0,4 % en acide acrylique.

Les tests d'absorption réalisés dans les mêmes conditions avec un anti-sérum anti-B montre que 95 à 100 %

de l'activité anti-B sont retrouvées dans les surnageants d'incubation.

EXEMPLE 5 : Fixation spécifique d'anti-corps anti-A sur une colonne de chromatographie.

On utilise un support PVC greffé à 2,7 % en acide acrylique sur lequel des stromas lyophilisés $A_1$, $R_1$ et $R_2$ sont fixés dans les conditions opératoires de l'exemple 3.

La colonne (diamètre 2,7 cm) est remplie avec 100 ml de support -stromas $A_1$ correspondant à environ 70 g de support humide sur lequel 3 g de stromas sont théoriquement fixés. Après équilibrage et lavages de la colonne au PBS, on introduit un échantillon constitué par 300 $cm^3$ d'albumine humaine, d'anti-A et d'anti-B titrant 1/32ème en technique manuelle tube centrifugation vis-à-vis de globules rouges $A_1$ et B.

A la sortie de colonne, on récupère intégralement l'activité anti-B et en fin de pic, une faible activité anti-A (titrant 1/4) correspondant à un excès d'activité anti-A déposé par rapport aux capacités de fixation de la colonne. Dans ces conditions opératoires 82 % de l'activité anti-A sont absorbés sur la colonne.

Le lavage de la colonne au PBS est poursuivi jusqu'à l'obtention d'une absorption nulle à 280 nm.

L'élution de l'anti-A fixé sur le support est obtenue à pH 2,8 en tampon glycine. L'éluat anti-A testé après neutralisation n'a pas d'activité anti-B.

EXEMPLE 6 : Purification d'une lectine de triticum vulgaris à l'aide d'un conjugué de l'invention.

On utilise une lectine de triticum vulgaris, c'est-à-dire de germe de blé (wheat germ agglutinin ou WGA).

Le conjugué mis en oeuvre est obtenu par couplage, selon le procédé de l'exemple 1 d'un support PCV greffé avec des motifs acrylamide puis soumis à une réaction d'hydrazinolyse à raison de 2,25 % avec un dérivé de N-acétyl-

glucosamine de formule :

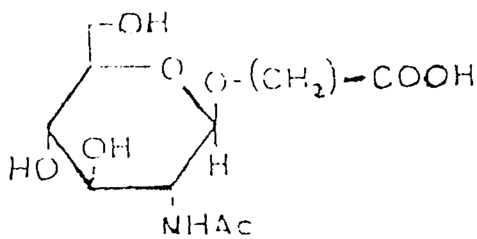

3

Sur une petite colonne contenant 1 $cm^3$ de conjugué activé, on dépose un échantillon de 0,3 ml de lectine brute de WGA. Après 5 minutes de contact à température ambiante, la colonne est lavée par du tampon NaCl, phosphate, pH 7,2, à un débit de 1 ml/mn.

Dans ces conditions, 69 % de l'activité WGA testée par agglutination des mématies humaines, sont fixés sur la colonne. Ceci correspond à 2,1 mg de protéine WGA fixée.

Par passage d'une solution de N-acétylglucosamine 0,1 M, à travers la colonne, on élue 95,5 % de l'activité fixée. L'activité restante est éluée par passage d'un tampon glycine 0,2 M, NaCl 0,15 M, pH 2,8.

Le complexe lectine WGA-N-acétylglucosamine est déplacé par dialyse exhaustive contre du tampon PBS, ce qui permet de récupérer la lectine sous une forme de pureté élevée.

EXEMPLE 7 : Purification de la lectine Ulex II.

On utilise le conjugué de l'exemple 6. Sur une colonne renfermant 1 $cm^3$ de conjugué, on dépose 6 ml d'un extrait aqueux brut d'Ulex Europaeus stabilisé à pH 6,6 et ayant un titre de 1/64 en tube à 22°C vis-à-vis d'hématies $A_1$.

Par rinçage de la colonne avec NaCl 0,15 M tamponné à pH 7,2, on récupère la fraction présentant un pic absorbant à 280 nm. Cette fraction possède une spécificité anti-H et correspond à la lectine Ulex I. Elle agglutine les globules rouges humains dans l'ordre d'agglutination

décroissante : O,B,$A_1$. Cette activité agglutinante est inhibée par le fucose.

Après passage d'une solution de NaCl 2M, la colonne est éluée par une solution de N-acétylglucosamine 0,1M puis avec un tampon glycine 0,2M, NaCl, 0,15M, pH 2,8. L'ensemble de ces deux éluats représente 7 mg de protéine. Ils agglutinent les hématies humaines indépendamment du groupe ABO. Une agglutination totale observée avec l'éluat à 0,75 mg/ml est complètement inhibée par la N-acétylglucosamine 0,125M finale.

Ces deux dernières fractions éluées représentent la lectine Ulex II.

Les exemples 6 et 7 montrent que toutes les lectines de spécificité $(N\text{-acétylglucosamine})_n$ peuvent être purifiées à l'aide des conjugués de l'invention, en particulier, les lectines de Solanum tuberosum (STA), de Laburnum alpinum, de Bandeirea simplicifolia (B S II).

EXEMPLE 8 : Préparation de conjugués hémocompatibles à partir d'héparine et d'un support de PVC comportant 3,1 % de groupes acrylamide fixés.

On procède tout d'abord à l'activation du support puis à la fixation d'héparine sur le support soit par l'intermédiaire d'un bras soit directement.

1 - Fixation d'héparine par l'intermédiaire d'un bras constitué par du diaminohexane.

. activation du support.

Après lavage et équilibrage du support avec un tampon phosphate 0,5 M pH 7,5, on ajoute aux fins d'activation du glutaraldéhyde à raison de 5 ml pour 5 g de support. Après agitation durant 16 heures à 37°C, on lave le support avec de l'eau jusqu'à l'obtention d'une densité optique égale à 0 à 280 nm.

Le dosage des fonctions activées donne une valeur de 364 uM par gramme de support sec.

. fixation du bras.

On ajoute 4 g de support sec et 8 ml de 1,6-diaminohexane à 20 mg/ml de tampon phosphate 0,5 M, pH 7,5, puis on soumet l'ensemble à agitation durant environ 14 heures à +4°C. Le mélange est filtré puis le support est lavé à l'aide du tampon ci-dessus, puis d'un tampon phosphate 0,05 M, contenant 0,5 M NaCl, pH 7,5.

Le taux de diaminohexane fixé est de 127 uM par gramme de support sec.

. condensation avec l'héparine du support avec le bras.

On ajoute au support précédemment obtenu, comportant le bras, 8 ml d'eau, 4 ml d'héparine à 20 mg/ml dans 420 et 100 mg de carboxyméthylcellulose.

Le pH est maintenu à 5,5-5 pendant 1 heure. Le mélange est soumis à agitation durant environ 14 heures à +4°C, puis filtré et lavé avec NaCl 1 M.

On obtient une fixation d'héparine de 5 mg par gramme de support.

2 - Fixation directe d'héparine.

On lave 3 g de support de PVC greffé avec 3 ml d'eau et 3 mg d'hydrazine.

Après une incubation de 1 heure à 50°C, on refroidit rapidement le mélange, puis on lave abondamment le support avec de l'eau. Aux fins de diazotation, on ajoute successivement HCl 0,5N puis on laisse sous agitation 5 minutes dans                                   puis on ajoute 70 mg/ml de nitrite de sodium en agitant ensuite 2 minutes.

Le mélange est filtré et le support ainsi activé est lavé.

. fixation d'héparine.

On ajoute 4 ml de solution d'héparine dans 50 mg/ml de tampon $NH_4$ Cl 3M pH 9,5 à l'équivalent de 2 g de support sec activé. Après agitation du mélange durant

environ 14 heures à +4°C, le conjugué obtenu est lavé à l'eau puis avec NaCl 0,5 N.

Le taux d'héparine fixée est de 12,85 mg par gramme de support sec.

. neutralisation des groupements actifs en excès.

On met en suspension le conjugué obtenu dans 2 ml d'éthanolamine 1 M, pH 8.

Après une agitation de 16 heures à 4°C du mélange, on filtre et on lave le conjugué avec de l'eau, puis un tampon phosphate 0,1 M pH 8,4 contenant 0,5 M NaCl, suivi d'un tampon acétate 0,1 M pH 4 contenant 0,5 M NaCl.

On conserve le conjugué dans du tampon phosphate 0,2 M pH 4,4 contenant 0,02 % d'azide de sodium.

EXEMPLE 9 : Détermination de supports hémocompatibles.

Des supports comportant des taux différents d'acide acrylique ou groupes acrylamide greffés ont été testés sur des sangs humains en utilisant divers tests de coagulation (Temps de thrombine, (TT), Temps de Howel, (TH), Temps de Céphaline Kaolin, (TCK), Thromboélastogramme sur plasma riche en plaquettes, (TEG/PRP), et numérations plaquettaires).

Les essais effectués avec les supports greffés à l'aide de motifs monomères acrylamide ont montré une variation des effets en fonction des taux de greffage, ainsi qu'il ressort du tableau ci-dessous. Les perturbations sont pratiquement négligeables pour des taux inférieurs à 10 % environ. L'augmentation des taux de greffage entraîne des modifications plus importantes.

| SANG N° 3 | PQ | T.T. | T.H. | T.C.K | TEG/PRP | | | |
|---|---|---|---|---|---|---|---|---|
| | | | | | r | k | r + k | amx |
| TEMOIN eau phys. | 211.000 | 23" | 2'30 | 56" | 31 | 10 | 41 | 60 |
| 22- Acide acrylamide 1 % | 210.000 | 24" | 2'30 | 56" | 27 | 8 | 35 | 60 |
| 25 - PVC Acide acrylamide 16 % | 219.000 | 27" | 2' | 56" | 25 | 11 | 36 | 58 |
| 21 - PVC Acide acrylamide 33 % | 194.000 | 34" | 2'15 | 56" | 13 | 3 | 16 | 69 |

EXEMPLE 10 : Préparation du dérivé de N-acétyl-glucosamine de formule 3.

On prépare ce dérivé par condensation, d'une part d'un dérivé d'oxazolidine de formule 4, elle-même obtenue selon la technique décrite par Lemieux et coll. dans J.A.C.S. (1975), volume 97, pp 4063-4069 à partir du chloro, 3,4,6-tri-O-acétyl-2-acétamido-2-désoxy-α-D-glucopyranoside (ce produit étant à son tour obtenu selon la technique décrite par Horton dans Methods in Carb. Chem. (1972), volume 4, pp 282-284) avec, d'autre part, un dérivé de l'acide azélaïque de formule 5, obtenu selon la méthode décrite par Lemieux et coll. dans J.A.C.S., (1975), volume 97, pp 4076-4083.

La condensation entre ces deux produits est effectuée selon une méthode modifiée par rapport à celle décrite par MATTA et coll. dans Carb. Research (1976), volume 51, pp 215-222, en procédant comme suit.

Sous atmosphère d'azote et conditions anhydres, on porte à reflux pendant 30 minutes, 2 mmoles du composé 4 et 2,5 mmoles du composé 5, en solution dans 8 ml d'un mélange anhydre de nitrométhane et de toluène (1:1) en présence de 25 mg d'acide p-toluène sulfonique sec.

L'analyse chromatographique est réalisée à l'aide de mélanges chloroforme:méthanol (20:1), acétate d'éthyle: hexane (8:2), et éther-méthanol (95:5).

A la fin de la réaction, on ajoute de la pyridine sèche, puis on concentre à sec.

Après solubilisation dans le chloroforme et plusieurs lavages à l'eau, on sèche la phase organique sur du sulfate de sodium, on concentre à sec, puis on purifie à l'aide de silice 60 G dans un mélange acétate d'éthyle-hexane (8:2). On obtient le produit de condensation de formule 6 avec un rendement de 35 %.

Afin d'obtenir le produit de formule 3 recherché,

on soumet le dérivé 6 à l'action de Na dans $CH_3OH$, ce qui permet de libérer les groupes hydroxyle et de transformer l'ester éthylique en ester méthylique, puis on soumet cet ester à l'action de NaOH en présence de méthanol, ce qui conduit à l'acide.

Ces deux dernières étapes sont effectuées selon la méthode décrite par Lemieux et coll. dans J.A.C.S. (1975), volume 97, pp 4076-4083.

Les formules dont question ci-dessus sont les suivantes :

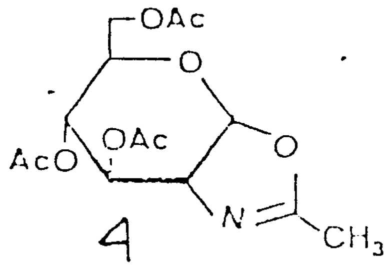

4

$HOCH_2-(CH_2)_7-COOEt$

5

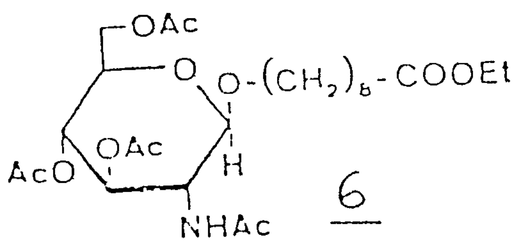

6

REVENDICATIONS

1.- Nouveaux conjugués caractérisés en ce qu'ils sont élaborés à partir de supports greffés et d'une molécule biologiquement active, fixée de façon covalente au support, cette molécule fixée renfermant au moins un motif saccharidique ou du type des glucosamino-glucuroglycanes et étant choisie en particulier parmi les déterminants antigéniques de groupe sanguin, l'héparine, des substrats biologiques, tels que des cellules ou des membranes cellulaires, en particulier, des stromas d'érythrocytes, la N-acétylglucosamine ou un dérivé de N-acétylglucosamine.

2.- Conjugués selon la revendication 1, caractérisés en ce que ladite molécule est fixée au support par l'intermédiaire d'une chaîne ou bras espaceur (1) formé à l'aide d'une part des motifs du monomère greffé sur ce support et d'autre part de la molécule en question et/ou (2) allongé par un composé chimique mis en oeuvre pour le couplage ou (3) fourni par ce composé.

3.- Conjugués selon la revendication 2, caractérisés en ce que le bras espaceur comporte un groupe hydrazide, ce groupe étant avantageusement obtenu directement à partir de supports greffés à l'aide de motifs monomères à fonction acrylamide, par transformation en fonction acrylazide.

4.- Conjugués selon l'une quelconque des revendications précédentes, caractérisés en ce que le taux de greffage des supports entrant dans la constitution des conjugués est inférieur à 10% environ, en particulier à 7% environ et de préférence de l'ordre de 1 à 5%, plus spécialement voisin de 2 à 3%.

5.- Conjugués selon l'une quelconque des revendications précédentes, caractérisés en ce que la

matière polymère de base formant le support est constituée par du polychlorure de vinyle (PVC) et que le support est avantageusement du type PVC-acrylamide ou PVC-acide acrylique.

6.- Conjugués selon la revendication 1, 4 ou 5, caractérisés en ce qu'ils renferment le déterminant antigénique B et répondent à la structure a

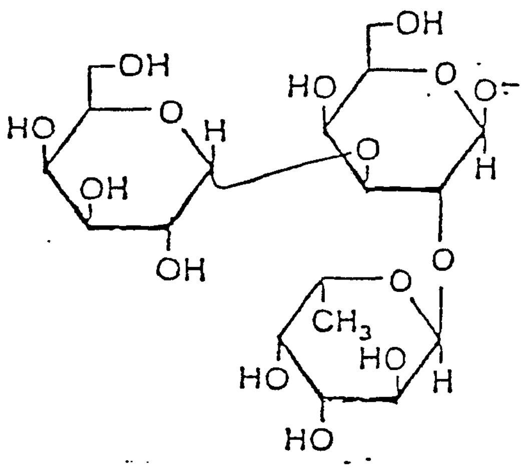 $(CH_2)_n$ - CO-NH-NH-CO-$(CH_2)_2$-X

avec n représentant un nombre de 2 à 8 et X représentant le restant du bras fourni par le support greffé et ce support de base lui-même.

7.- Conjugués selon la revendication 6, caractérisés en ce qu'ils comportent le déterminant antigénique A à la place du déterminant B.

8.- Conjugués selon l'une quelconque des revendications 1, 4 ou 5, caractérisés en ce que ladite molécule fixée est constituée par l'héparine, cette dernière étant fixée sur le support directement ou par l'intermédiaire d'un bras, notamment un bras du type diaminohexane.

9.- Conjugués selon l'une quelconque des revendications 1,4 ou 5, caractérisés en ce qu'ils répondent à la structure b

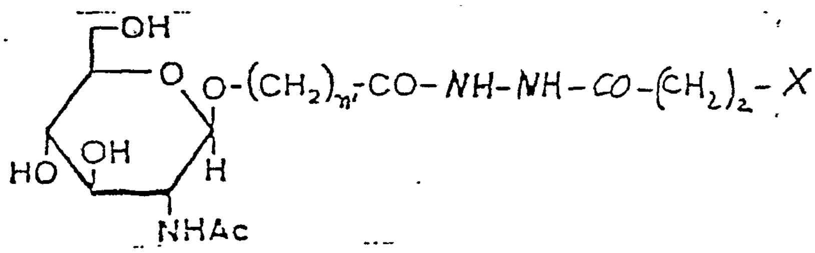

dans laquelle n'est un nombre de 2 à 8, Ac représente le groupe acétyle et X est tel que défini ci-dessus.

10. Procédé de préparation de conjugués selon l'une quelconque des revendications 1 à 9, caractérisés par la mise en contact d'une molécule à activité biologique avec le support greffé, avantageusement activé selon les techniques classiques, et le cas échéant, traité au préalable afin de modifier les groupes fonctionnels en bout de chaîne.

11.- Procédé selon la revendication 10, caractérisé en ce que pour préparer les conjugués renfermant un déterminant antigénique de groupe sanguin, on met en oeuvre (1) un support greffé à l'aide de motifs acrylamide, puis soumis à une hydrazinolyse afin de former des groupements acrylhydrazide et (2) un dérivé du déterminant antigénique comprenant une chaîne terminée par une fonction carboxyle, de formule $-O-(CH_2)_n-COOH$, dans laquelle n est avantageusement un nombre de 2 à 4, les groupes -OH du déterminant antigénique étant bloqués par des groupes protecteurs.

12.- Procédé selon la revendication 11, caractérisé en ce que le dérivé du déterminant antigénique est au préalable activé et qu'on effectue le couplage de manière progressive sous agitation, de préférence à température ambiante, dans des solvants du type du diméthylformamide.

13.- Procédé selon l'une quelconque des revendications 10 à 12, caractérisé en ce qu'on utilise des quantités

équimolaires de déterminant antigénique de N-méthyl morpholine et de chloroformiate d'alkyle.

14.- Procédé selon la revendication 12 ou 13, caractérisé en ce qu'on utilise un volume de milieu d'activation équivalent à celui du support humide et que, de préférence, on soumet le support hydrazidé à un dégazage avant d'effectuer le couplage.

15.- Procédé selon la revendication 11, caractérisé en ce que le dérivé du déterminant antigénique (2) est obtenu à partir du dérivé correspondant (3) dans lequel tous les groupes -OH sont bloqués par des groupements protecteurs et la chaîne de substitution est constituée par un groupe $-O-(CH_2)_{n-1}-OH$, qu'on soumet ce dérivé (3) à un traitement d'oxydation, avantageusement à l'aide d'un agent oxydant tel que l'anhydride chromique, en opérant à température ambiante,dans un milieu solvant du type de ceux renfermant en mélange le dichlorométhane et la pyridine, qu'on transforme la terminaison aldéhyde du dérivé intermédiaire en chaîne $-O-(CH_2)-CH=CH-COOMe$ sous l'action d'un traitement du type de celui effectué à l'aide du réactif de Wittig, avantageusement à une température de l'ordre de 100°C, notamment de 90°C, puis qu'on effectue une étape d'hydrogénation, avantageusement en présence de catalyseur, et qu'on saponifie l'ester méthylique formé.

16.- Procédé selon la revendication 10, caractérisé en ce que les conjugués renfermant des stromas sont préparés par mise en contact (1) d'un support greffé préalablement lavé et activé par le glutaraldéhyde et (2) des stromas, également soumis à un prélavage, le couplage étant de préférence réalisé à base température, plus spécialement entre 0 et 10°C, environ, avantageusement à environ 4°C.

17.- Procédé selon la revendication 10, caractérisé en ce que les conjugués renfermant un dérivé de N-acétyl

glucosamine sont obtenus à partir d'un support greffé à l'aide de motifs acrylamide avantageusement à raison d'environ moins de 10%, notamment de 1 à 7% ces motifs étant soumis à un traitement d'hydrazinolyse afin de former des groupes acrylhydrazide et d'un dérivé de N-acétyl glucosamine comportant un bras de substitution, avantageusement en position 1, à terminaison carboxyle.

18.- Application des conjugués selon l'une quelconque des revendications 1 à 9, pour des épurations thérapeutiques ou comme réactifs biologiques dans le cas de conjugués ayant une activité de groupe sanguin ou ceux comportant des oligosaccharides.

19.- Produits intermédiaires caractérisés en ce qu'il s'agit des déterminants antigéniques A et B de groupe sanguin, comportant, sur le carbone anomère du motif galactopyranose lié au motif fucopyranose, une chaîne de substitution à terminaison carboxyle du type $-O-(CH_2)_n-COOH$ avec $\underline{n}$ représentant un nombre de 1 à 4.